(19) Europäisches Patentamt
European Patent Office
Office européen des brevets

(11) Publication number: 0 224 332 B1

(12) # EUROPEAN PATENT SPECIFICATION

(45) Date of publication of patent specification: 08.08.90

(21) Application number: 86308208.7

(22) Date of filing: 22.10.86

(51) Int. Cl.[5]: C 07 D 491/052, C 07 D 311/58, A 61 K 31/40 // (C07D491/052, 311:00, 209:00)

(54) Heterocyclic amino compounds.

(30) Priority: 23.10.85 GB 8526210

(43) Date of publication of application: 03.06.87 Bulletin 87/23

(45) Publication of the grant of the patent: 08.08.90 Bulletin 90/32

(84) Designated Contracting States: AT BE CH DE ES FR GB GR IT LI LU NL SE

(56) References cited:
EP-A-0 162 592
EP-A-0 162 593
FR-A-2 534 921

CHEMICAL ABSTRACTS, vol. 56, no. 12, 11 June 1962, Columbus, OH (US); A.FUNKE et al.: "Benzo-1,4-dioxanes disubstitutedin positions 2, 3", nos. 14257i-14258b

CHEMICAL ABSTRACTS, vol. 103, no. 15, 14 Oct 1985, Columbus, OH (US); "Chromandicarboximide derivatives", p. 716, no. 123358a

(73) Proprietor: GLAXO GROUP LIMITED
Clarges House 6-12 Clarges Street
London W1Y 8DH (GB)

(72) Inventor: Kitchin, John
68 Boldmere Road Eastcote
Pinner Middlesex HA5 1PP (GB)
Inventor: Cherry, Peter Clive
Thimble Lodge Lane
Chalfont St.Giles Bucks HP8 4AQ (GB)
Inventor: Borthwick, Alan David
15 Temple Grove
London NW11 7UA (GB)
Inventor: Pipe, Adrian John
30 Rugby Avenue
Greenford, Middlesex UB6 0EY (GB)
Inventor: Burn, Derek
77 Sycamore Road
Chalfont St. Giles Buckinghamshire (GB)
Inventor: Coles, Richard John
28 Norfolk Road
Uxbridge Middlesex (GB)

(74) Representative: Kyle, Diana et al
ELKINGTON AND FIFE Beacon House 113 Kingsway
London WC2B 6PP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 224 332 B1

Courier Press, Leamington Spa, England.

**Description**

This invention relates to heterocyclic amino compounds. More specifically this invention relates to novel benzopyranopyrrole derivatives, to processes for the preparation thereof, to pharmaceutical preparations containing them, and to their use in medicine.

The alpha (α)-adrenoreceptors of the sympathetic nervous system are classified pharmacologically into two sub-groups, namely $\alpha_1$ and $\alpha_2$. The $\alpha_2$-type are situated predominantly on the presynaptic terminals of noradrenergic neurones and are activated by the released neurotransmitter. Such activation results in a diminished release of noradrenaline on subsequent stimulation of the neurones, the $\alpha_2$-adrenoreceptors thus forming part of an autoinhibitory feedback mechanism for regulating the synaptic concentration of the neutrotransmitter. A selective $\alpha_2$-adrenoreceptor anatagonist would be expected to produce an increase in the synaoptic concentrations of noradrenaline by blocking the autoinhibitory feedback mechanism and would thus be of potential value in human medicine for the treatment of disorders such as depression which are associated with a deficiency of noradrenaline at postsynaptic adrenoreceptors.

$\alpha_2$-Adrenoreceptors also occur at non-neuronal sites such as on blood-platelets, in pancreatic islet cells, on adipocytes and in the proximal tubules of the kidney. Activation off $\alpha_2$-adrenoreceptors at these sites leads to platelet aggregation, inhibition of insulin release, inhibition of lipolysis and retention of sodium respectively.

A selective $\alpha_2$-adrenoreceptor antagonist thus has a potential therapeutic use as an antidepressant either alone or in a complimentary combination with an established antidepressant, and in either treating or preventing conditions such as migraine, thrombosis, diabetes, obesity, hypertension, constipation, paralytic ileus and senile dementia.

We have now found that the compounds of formula (I) below and their physiologically acceptable salts have a selective $\alpha_2$-adrenoreceptor antagonist action.

The invention thus provides compounds of general formula (I)

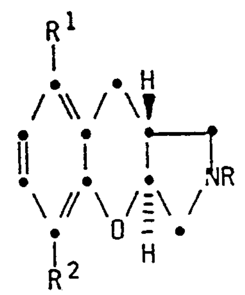

(I)

wherein

R is a hydrogen atom or a group selected from $C_{1-6}$ alkyl (optionally substituted by $C_{3-7}$ cycloalkyl), $C_{3-6}$ alkenyl, $C_{3-6}$ alkynyl, $C_{3-7}$ cycloalkyl, and phen $C_{1-5}$ alkyl; and

$R^1$ and $R^2$, which may be the same or different, represent a hydrogen atom or a fluorine or chlorine atom provided that $R^1$ and $R^2$ do not both represent a hydrogen atom;

and the physiologically acceptable salts thereof.

In general formula (I), the alkyl, alkenyl and alkynyl groups represented by R may be straight or branched chain groups.

When R contains a —C=C— or —C≡C— linkage this is not directly attached to the nitrogen atom. When R is alkyl it may be, for example, methyl, ethyl or propyl, methyl being preferred. When R is an alkyl group substituted by a $C_{3-7}$ cycloalkyl group it may be, for example, cyclopropyl $C_{1-3}$ alkyl such as cyclopropylmethyl. When R is alkenyl it may be, for example, allyl and when R is akynyl it may be, for example, cyclopropyl. When R is cycloalkyl it may be, for example, cyclopropyl. When R is phen$C_{1-5}$ alkyl it may be, for example, benzyl.

Suitable physiologically acceptable salts are the acid addition salts formed with inorganic acids, for example hydrochlorides, hydrobromides, phosphates and sulphates, and with organic acids, for example citrates, tartrates, acetates, maleates and succinates. The hydrochlorides are particularly useful.

It will be appreciated that each compound of general formula (I) is a *trans* isomer and exists as two enantiomers. The structural formulae herein are to be understood to depict either enantiomer of each compound concerned as well as mixtures of the enantiomers, including racemates, even though the precise structure as set out only relates to one enantiomer.

A preferred group of compounds of general formula (I) is that wherein R is a hydrogen atom. Another preferred group of compounds of general formula (I) is that wherein R is a $C_{1-3}$ alkyl group, particularly a methyl or ethyl group. A further preferred group of compounds of general formula (I) is that wherein $R^1$ and/or $R^2$ represent a fluorine atom.

A particularly preferred group of compounds of formula (I) are those in which R is a hydrogen atom, and one of $R^1$ and $R^2$ is a hydrogen atom and the other represents a fluorine atom.

Important compounds are (±)-*trans*-5-fluoro-1,2,3,3a,9,9a-hexahydro[1]benzopyrano[2,3-c]pyrrole, and its 3aS- and 3aR-isomers; (±)-*trans*-8-fluoro-1,2,3,3a,9,9a-hexahydro[1]benzopyrano[2,3-c]pyrrole, and its 3aS- and 3aR-isomers; (±)-*trans*-8-fluoro-1,2,3,3a,9,9a-hexahydro[1]benzopyrano[2,3-c]pyrrole, and its 3aS- and 3aR- isomers; and their physiologically acceptable salts, particularly the hydrochlorides.

The compounds of the invention have selective $\alpha_2$-adrenoreceptor antagonist action. The test for determining the $\alpha_2$-adrenoreceptor antagonist action is based on the ability to prevent the action of a selective $\alpha_2$-adrenoreceptor agonist such as clonidine or 5-bromo-N-(4,5-dihydro-1H-imidazol-2-yl)-6-quinoxalanamine-[R-(R*R*)]-2,3-dihydroxy-butanedioate (UK 14304—18) on the rat field stimulated vas deferens preparation.

The compounds of the invention are thus of interest in the treatment of prevention of migraine, thrombosis, diabetes, obesity, hypertension, constipation, paralytic ileus and senile dementia, and in particular for the treatment of depression.

The invention accordingly further provides compounds of general formula (I) and their physiologically acceptable salts for use in the therapy or prophylaxis of migraine, thrombosis, diabetes, obesity, hypertension, constipation, paralytic ileus and senile dementia and in particular depression. The compounds of the invention may be used either alone or with an additional active ingredient. Thus, for example, in the treatment of depression, the compound of the invention may be used alone, or may be co-administered with an established antidepressant (e.g. desmethylimipramine imipramine or amitriptyline) either in a single formulation or in separate formulations. The established antidepressant can be used in accordance with conventional practice.

The compounds according to the invention may be formulated in a conventional manner, optionally together with one or more other active ingredient, for administration by any convenient route for example for oral, rectal, intravenous or intramuscular administration. Oral administration is preferred.

Thus according to another aspect, the invention provides a pharmaceutical composition comprising a compound of general formula (I) and/or a physiologically acceptable salt thereof together with a physiologically acceptable carrier or excipient. The composition may optionally contain an additional active ingredient, for example an antidepressant such as desmethylimipramine, imipramine or amitriptyline.

For oral administration, the pharmaceutical composition may take the form of, for example, tablets, capsules, powders, solutions, syrups or suspensions prepared by conventional means with physiologically acceptable excipients, including controlled release formulations.

Compositions for rectal administration may be in the form of suppositories using a conventional suppository excipient.

The compounds may be formulated for intravenous or intramuscular administration in dry form for reconstitution before use, or as a sterile solution or suspension.

A proposed daily dose for administration to man is 0.002 to 10 mg/kg, for example 0.01 to to 10 mg/kg, preferably 0.01 to 3 mg/kg, which may be conveniently administered in 1 to 3 doses per day. The precise dose administered will of course depend on the age and condition of the patient. The daily dosage may conveniently be administered in the form of dosage units, each unit containing for example 0.01 to 3 mg/kg, preferably 0.002 to 3 mg/kg, of active ingredient.

The compounds according to the invention may be prepared by a number of processes. In the following description the groups R, $R^1$ and $R^2$ are as previously defined for general formula (I) except where otherwise indicated.

According to a first example (A), compounds of general formula (I) may be prepared by amination of a compound of general formula (II)

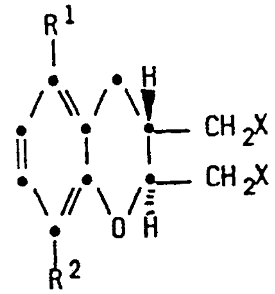

(II)

[where X is a leaving group such as a halogen atom, (e.g. chlorine, bromine or iodine), or a hydrocarbylsulphonyloxy group (e.g. methylsulphonyloxy or p-toluenesulphonyloxy)] with ammonia, aqueous ammonia or an amine of formula $RNH_2$ where R is as previously defined.

In a particular embodiment of this process, following the amination reaction, the resulting compound of general formula (I) or a salt thereof, may be converted into another compound of general formula (I). Thus, for example, when R is arylmethyl, the amination reaction may optionally be followed by removal of the arylmethyl group to yield a compound of formula (I) where R is a hydrogen atom.

The amination reaction is conveniently effected at an elevated temperature e.g. at 120°C, optionally in the presence of a suitable base e.g. sodium hydride or an alkali metal hydroxide such as sodium hydroxide

or preferably in the presence of an excess of the amine $RNH_2$. The reaction may be carried out in the absence or presence of a solvent such as an ether e.g. dioxan, chlorinated hydrocarbon e.g. chloroform or an alcohol e.g. ethanol. Optional removal of an arylmethyl group may be carried out for example by hydrogenolysis or, where appropriate, under acidic conditions, as described below.

According to another example (B), a compound of general formula (I) where R represents a hydrogen atom may be prepared by deprotection of a corresponding compound where R represents a protecting group. Suitable protecting groups include, for example, arylmethyl and acyl groups. Conventional deprotection procedures may be used. For example, where appropriate an arylmethyl group (e.g. benzyl) may be removed by hydrogenolysis using, for example, hydrogen in the presence of a catalyst, such as platinum or palladium on a support (e.g. charcoal), in a solvent such as an alcohol e.g. methanol. Alternatively, where appropriate, an arylmethyl group (e.g. trityl) may be removed under acidic conditions, using for example an acid such as trifluoroacetic acid, formic acid or hydrobromic acid. Acyl groups may be removed by hydrolysis using an acid such as mineral acid or a base such as an alkali metal hydroxide as appropriate. The protected starting materials for this process may be prepared using standard methods for the protection of amines, for example as described by J. F. W. McOmie in 'Protective Groups in Organic Chemistry' (Plenum Press, 1973).

The product of either of the processes (A) or (B) described above may be subjected to one or two further reactions comprising: (C)(i) converting the resulting compound of general formula (I) or a salt thereof into another compound of general formula (I); and/or (C)(ii) converting a compound of general formula (I) or a salt thereof into a physiologically acceptable salt thereof.

It is also possible to prepare a compound of general formula (I) by a process comprising interconversion of another compound of general formula (I).

For example, a compound of general formula (I) in which R is a hydrogen atom may be converted by alkylation to a compound of general formula (I) in which R is an alkyl, substituted alkyl, alkenyl, alkynyl or aralkyl group. Conventional alkylation procedures may be used, for example reductive alkylation using an appropriate aldehyde with a complex metal hydride such as sodium or potassium borohydride or sodium cyanoborohydride in a suitable solvent such as an alcohol e.g. methanol.

Alternatively, the alkylation may be performed with an alkylating agent RX (where R is an alkyl, substituted alkyl, alkenyl, alkynyl or aralkyl group and X is a leaving group such as a halogen atom e.g. chlorine or bromine, or a hydrocarbylsulphonyloxy group, e.g. *p*-toluenesulphonyloxy) preferably in the presence of a base, such as potassium carbonate, optionally in a solvent such as an alcohol, e.g. ethanol.

Physiologically acceptable salts of the compounds of general formula (I) may be prepared by reacting the free base of formula (I) or a salt thereof with an appropriate acid, such as hydrogen chloride in the presence of a suitable solvent e.g. an ester such as ethyl acetate, an alcohol such as ethanol, ether or dichloromethane to obtain the desired physiologically acceptable salt.

In the following description relating to the preparation of intermediates, where enantiomers exist, the structural formulae herein are to be understood to depict either enantiomer as well as mixtures, including racemates, although the precise structure as set out only relates to one enantiomer.

The intermediate compounds of general formula (II) may be prepared by reaction of a corresponding diol of formula (III)

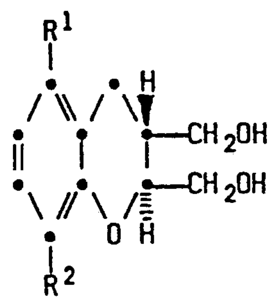

(III)

with a halide of formula $X_1A$ (where $X_1$ is a hydrocarbylsulphonyl group e.g. methylsulphobyl and A is a halogen atom e.g. a chlorine atom) in the presence of a base e.g. triethylamine in a solvent such as dichloromethane; or with a halogenating agent such as thionyl chloride, phosphorous tribromide or hydrogen iodide.

The diols of formula (III) may be prepared by the following sequence of reactions;

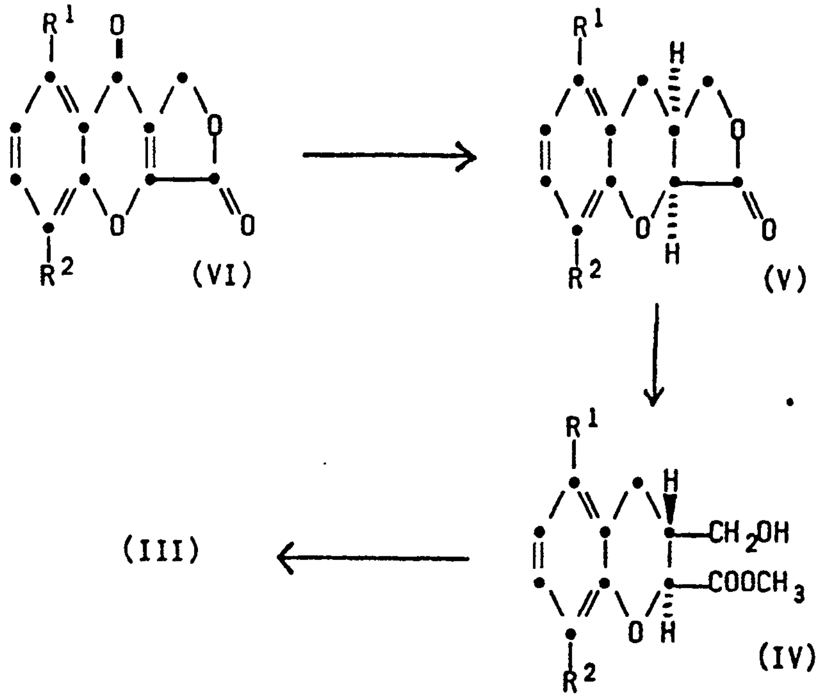

(VI) (V) (IV) (III)

Reduction of the pyrone (VI) using hydrogen and palladium on charcoal in acetic acid or in a solvent such as ethyl acetate or dioxan containing acetic acid yields the lactone (V), which is converted to the ester (IV) by reaction with potassium carbonate and methanol. Reduction of the ester (IV) using lithium aluminium hydride in tetrahydrofuran then gives the required diol (III).

The intermediate pyrone of formula (VI) may be prepared from a compound of formula (VII)

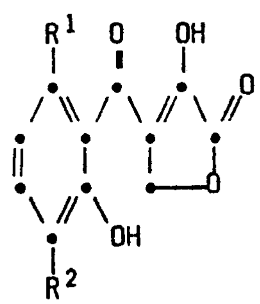

(VII)

The reaction may be carried out under acidic conditions preferably with heating in a solvent such as glacial acetic acid. Suitable acids for the reaction include mineral acids eg hydrochloric acid or p-toluenesulphonic acid.

The compound (VII) may be prepared from a compound of formula (VIII)

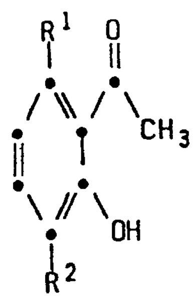

(VIII)

by reaction with diethyl oxalate in the presence of a strong base such as an alkali metal alkoxide eg sodium

ethoxide in a solvent eg an alcohol such as ethanol preferably with heating eg at reflux, followed by subsequent reaction of the product formed with aqueous formaldehyde.

Intermediates of formula (VIII) in which $R^2$ represents a fluorine or chlorine atom and $R^1$ is as defined in formula (I) are either known compounds or can be prepared according to the methods described by J. A. Donnelly and J. J. Murphy, J. Chem. Soc. (C), 1970, 2596—2598.

Intermediates of formula (VIII) in which $R^2$ represents hydrogen and $R^1$ is a fluorine atom may be prepared by the following reaction sequence:

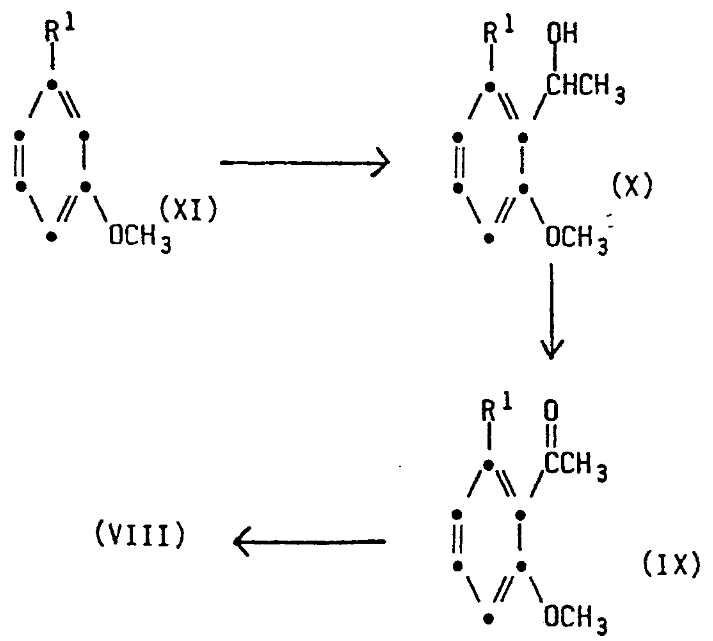

(XI) (X) (IX) (VIII)

Reaction of the known haloanisole (XI) with a strong base such as n-butyl lithium at low temperatures eg −75°C, and subsequent addition of acetaldehyde, yields the compound (X), which is oxidised to the ketone (IX) using a suitable oxidising agent such as pyridinium chlorochromate. Demethylation of the ketone (IX) under standard conditions, eg using boron tribromide in a halohydrocarbon solvent such as dichloromethane provides the required intermediates (VIII).

Intermediate diols of formula (III) are novel compounds and constitute a further aspect of the present invention.

A specific enantiomer of general formula (I) may be prepared by resolution of a mixture of enantiomers of formula (I) by conventional methods, e.g. by salt formation with an optically active acid followed by separation of the resulting diastereoisomeric salts, e.g. by fractional crystallisation. Alternatively, resolution may be effected at any suitable intermediate stage.

The following examples illustrate the invention. All temperatures are in °C. 'Dried' refers to drying using magnesium sulphate unless otherwise stated. Chromatography was carried out using silica gel. THF denotes tetrahydrofuran.

Intermediate 1

2-Fluoro-6-methoxy-α-methyl benzenemethanol.

n-Butyl lithium (3.9 mls) of 1.4 M in hexane) was added dropwise to a solution of 3-fluoroanisole (0.45 mls) in dry THF (10 mls) at −75° under nitrogen. The solution was kept at −75° for 1 hour, and then acetaldehyde (0.68 mls) was added dropwise and the mixture allowed to warm up. 2M Hydrochloric acid (20 mls) was added, the mixture was extracted with ether, the organic solution was dried and the solvent was evaporated. The product was distilled to give the *title compound* as an oil (0.4 g). NMR ($CDCl_3$) δ 7.17 (1H, d of t, 4-H), 6.75—6.6 (2H, m, 3-H, 5-H), 5.24 (1H, m, CHO—), 3.89 (3H, s, $OCH_3$), 3.27 (1H, d, OH), 1.56 (3H, d, $CH_3$).

Intermediate 2

1-(2-Fluoro-6-methoxyphenyl)ethanone.

Pyridinium chlorochromate (47 g) was added in portions to a stirred solution of Intermediate 1 (25 g) in dichloromethane (200 mls). After 3 hours, a further 10 g of reagent was added and stirring was continued overnight. The solution was decanted, the residue was washed thoroughly with ether (200 mls), and the combined solutions were washed through a short column of silica gel, using ether as solvent. Evaporation of the solvent gave a liquid (24 g). A portion was distilled to yield *title ketone* (10.4 g). NMR ($CDCl_3$) δ 7.30 (1H, d of t, 4-H), 6.77—6.65 (2H, m, 3-H, 5-H), 3.85 (3H, s, $OCH_3$), 2.53 (3H, s, $CH_3$).

Intermediate 3

1-(2-Fluoro-6-hydroxyphenyl)ethanone.

Boron tribromide (7.3 mls) was added to a stirred solution of Intermediate 2 (12.6 g) in dichloromethane (80 mls) at −65°, and the mixture was allowed to warm to 20° over 2 hours. Water (50 mls) was added dropwise to the stirred mixture, while cooling in a water bath. The layers were separated, the aqueous layer was extracted with dichloromethane and the combined organic solutions were dried (phase separating paper) and the solvent was evaporated. The resulting oil was distilled under vacuum to give the *title compound* as an oil (6.5 g). NMR ($CDCl_3$) δ 12.71 (1H, s, OH), 7.36 (1H, d of t, 4-H), 6.85—6.4 (2H, m, 3-H, 5-H), 2.69 (3H, d, $CH_3$).

Intermediate 4

4-[(3-Fluoro-2-hydroxyphenyl)carbonyl]-3-hydroxy-2(5H)-furanone.

A solution of 1-(3-fluoro-2-hydroxyphenyl)ethanone (30 g) and diethyl oxalate (31 g) in ethanol (600 mls) was added dropwise to sodium ethoxide (from 9.85 g of sodium) in ethanol (600 mls) and the mixture was stirred and heated at reflux for 2 days. The mixture was cooled, diluted with 60—80 petrol and the solid was collected and dried. A suspension of the product in water (1.2 l) was stirred with formaldehyde (24 mls of 37%) for 1.5 hours. The solid was filtered out, the filtrate was washed with ether, and traces of ether were removed by evaporation under vacuum. The aqueous solution was acidified with pH 3, the precipitate was collected, washed with water and recrystallised from acetone/water to give the *title compound* as yellow needles (18.7 g). M.p. 194—208° (decomp), i.r. $\nu_{max}$ (nujol) 1768, 1642 $cm^{-1}$ (C=O).

Intermediate 5

5-Fluoro-1H-furo[3,4-b][1]benzopyran-3,9-dione.

A solution of Intermediate 4 (24 g) in acetic acid (200 mls) and concentrated hydrochloric acid (40 mls) was heated at reflux for 1 hour. The mixture was allowed to cool, and was concentrated to a volume of 100 mls. Water was added, the precipitate solid was collected, washed with water and dried to yield the *title compound*. M.p. 164°.

Intermediate 6

Cis-(±)-5-Fluoro-9,9a-Dihydro-1H-furo[3,4-b][1]benzopyran-3(3aH)-one.

Intermediate 5 (23 g) in 1,4-dioxan (500 mls) was hydrogenated for 5 days over 10% palladium on carbon (3 g). Acetic acid (10 mls) and a further 0.8 g of catalyst were added, and hydrogenation was continued for a further 23 hours. The catalyst was filtered off, washed with ethyl acetate and the filtrate was concentrated to a volume of 150 mls. The suspension was filtered, the filtrate was evaporated to dryness, and the resulting gum was purified by chromatography eluting with dichloromethane to give the *title compound* as a white solid. M.p. 75.5—77.5°.

Intermediate 7

trans-(±)-8-Fluoro-3,4-dihydro-3-(hydroxymethyl)-2H-1-benzopyran-2-carboxylic acid methyl ester.

A mixture of Intermediate 6 (2 g) and dried potassium carbonate (3.9 g) in dry methanol (20 mls) was stirred under nitrogen for 23 hours. Wet ethyl acetate was added, and the mixture was stirred for 20 minutes. Further quantities of ethyl acetate and water were added, the ethyl acetate layer was separated, washed with water, dried and the solvent was evaporated. The resulting oil (0.58 g) was purified by chromatography eluting with dichloromethane-methanol (15:1) to yield the *title compound* as an oil. NMR ($CDCl_3$) δ 7.0—6.72 (3H, m, aromatic), 4.96 (1H, d, 2-H), 3.87 (3H, s, methyl ester), 3.67 (2H, d, $CH_2O$—), 2.9—2.5 (3H, m, 3-H, 4-$H_2$), 2.15 (1H, Br, OH).

Intermediate 8

trans-(±)-8-Fluoro-3,4-dihydro-2H-1-benzopyran-2,3-dimethanol.

A solution of Intermediate 7 (2.2 g) in dry THF (30 mls) was added dropwise to a stirred suspension of lithium aluminium hydride (0.46 g) in THF (30 mls) at 0—5°. After 1 hour, saturated ammonium chloride solution (10 mls) was added, the solid was filtered off and washed with THF. Evaporation of the combined solutions yielded a gum which was partitioned between ethyl acetate and water. The aqueous layer was evaporated to dryness, the residue was extracted with ethyl acetate and the combined ethyl acetate layers were dried and the solvent was evaporated. A solution of the product in a small volume of ethyl acetate was diluted with petrol until no nore solid precipitated. The solid was collected and dried to yield the *title compound* (1.5 g), M.p. 111°.

Intermediate 9

trans-(±)-5-Fluoro-3,4-dihydro-2H-1-benzopyran-2,3-dimethanol.

The *title compound*, m.p. 99—101°, was prepared from 1-(2-fluoro-6-hydroxyphenyl)ethanone (the product of Intermediate 3) by a sequence of reactions analogous to those described in Intermediates 4, 5, 6, 7 and 8.

Intermediate 10

trans-(±)-8-Fluoro-3,4-dihydro-2H-1-benzopyran-2,3-dimethanol, bis (methanesulphonate).

Methanesulphonyl chloride (1.4 g) was added dropwise to a stirred suspension of Intermediate 8 (1.4 g) in dichloromethane containing triethylamine (1.6 g) at 0—5°. After 1.5 hours the mixture was washed with 2M hydrochloric acid, the acid layer was extracted with dichloromethane, and the combined organic solutions were dried (phase separating paper) and evaporated to dryness. The residue was purified by column chromatography eluting with dichloromethane-methanol (20:1) to yield the *title compound* as a gum (2.4 g). NMR ($CDCl_3$) δ 7.0—6.75 (3H, m, aromatics), 4.54 (2H, d, 2-$CH_2O$, 4.42—4.22 (3H, m, 3—$CH_2O$, 2-H), 3.10, 3.05 (6H, s, s, $CH_3$), 3.02—2.76 (2H, m, 4-$H_2$), 2.53 (1H, m, 3-H).

Intermediate 11

trans-(±)-5-Fluoro-3,4-dihydro-2H-1-benzopyran-2,3-dimethanol bis (methanesulphonate).

A solution of methanesulphonyl chloride (0.77 mls) in dichloromethane (10 mls) was added dropwise to a stirred solution of Intermediate 9 (0.96 g) and triethylamine (1.9 mls) in dichloromethane (10 mls) at 0°. After 1 hour the solution was washed successively with 2M hydrochloric acid and sodium bicarbonate solution. The organic phase was dried (phase separating paper) and the solvent was evaporated. The resulting solid was triturated with ether, and recrystallised from toluene/cyclohexane to give the *title compound* as white crystals (0.7 g). M.p. 116—118°.

Example 1

trans-(±)-8-Fluoro-1,2,3,3a,9,9a-hexahydro-2-(phenylmethyl)-[1]benzopyrano[2,3-c]pyrrole.

A mixture of Intermediate 11 (0.63 g) and benzylamine (0.93 mls) was heated at 120°C under nitrogen for 45 minutes. Water (10 mls) was added to the cold mixture to give a solid which was collected, washed with water and dried to give the *title compound* (0.43 g), t.l.c. (petroleum ether b.p. 60—80°—ethyl acetate 1:1) Rf 0.65, NMR ($CDCl_3$) δ 7.4—7.2 (5H, m, Ph), 7.06 (1H, q, 6-H), 6.7—6.58 (2H, m, 5-H, 7-H), 4.05 (1H, d of t, 3a—H), 3.88, 3.79 (2H, ABq, $CH_2$Ph), 3,18, 3.0 (each 2H, t, 1-$H_2$, 3-$H_2$), 2.67—2.42 (2H, m, 9-$H_2$), 2.29 (1H, m, 9a-H).

Example 2

trans-(±)-8-Fluoro-1,2,3,3a,9,9a-hexahydro[1]benzoyrano[2,3-c]pyrrole hydrochloride.

A solution of Example 1 (0.42 g) in methanol (10 mls) was treated with excess hydrogen chloride in methanol and the solution was evaporated to dryness. The residue was redissolved in methanol and was hydrogenated over 10% palladium on charcoal (50 mg) at 40—50° until reaction was complete. The solvent was evaporated, the product was treated with hydrogen chloride in ethanol, evaporated to dryness and the solid was triturated with isopropyl alcohol to give the *title compound* (0.25 g). M.p. 220—2°. NMR (DMSO$d_6$) δ 9.72 (2H, Br-s, $NH_2^+$), 7.27 (1H, q, 6-H), 6.9—6.7 (2H, m, 5-H, 7-H), 4.21 (1H, d of t, 3a-H), 3.78—2.6 (6H, m, 3-$H_2$, 1-$H_2$, 9-$H_2$), 2.22 (1H, m, 9a-H).

Example 3

trans-(±)-5-Fluoro-1,2,3,3a,9,9a-hexahydro-2-(phenylmethyl)-[1]benzopyrano[2,3-c]pyrrole, hydrochloride.

A mixture of benzylamine (6.7 g) and Intermediate 10 (2.3 g) was stirred and heated to 120° under nitrogen for 50 minutes. After cooling, the mixture was partitioned between ethyl acetate and 2M sodium hydroxide solution, the layers were separated and the aqueous layer was further extracted with ethyl acetate. The combined organic solutions were washed with brine and then shaken with 2M hydrochloric acid. The precipitated solid was collected, washed with water and ethyl acetate, and dried to give the *title compound* as a white powder (1.3 g). M.p. 210—212°. NMR (DMSO$d_6$) δ 12.28, 12.00 (1H, 2×Br, S, $NH^+$), 7.8—7.6, 7.6—7.4 (5H, m, Ph), 7.2—6.85 (3H, m, 7-H, 6-H, 8-H), 4.65—4.1 (3H, m, $CH_2$Ph, 3a-H), 3.9—2.25 (7H, m, 1-$H_2$, 3-$H_2$, 9-$H_2$ 9a-H).

Example 4

trans-(±)-5-Fluoro-1,2,3,3a,9,9a-hexahydro[1]benzopyrano[2,3-c]pyrrole hydrochloride.

A solution of Example 3 (1.2 g) in methanol (50 mls) was hydrogenated over 10% palladium on charcoal (0.2 g) for 17 hours. The catalyst was filtered off, and the filtrate was evaporated to dryness. Crystallisation of the resulting solid from isopropyl alcohol/methanol gave the *title compound* (0.63 g). M.p. 259°. NMR (DMSO$d_6$) δ 9.9 (2H, Br S, $NH_2^+$), 7.2—6.85 (3H, m, aromatic), 4.24 (1H, d of t, 3a-H), 3.8—2.78 (6H, m, 1-$H_2$, 3—$H_2$, 9-$H_2$), 2.27 (1H, m, 9a-H).

Pharmaceutical Examples

In the following examples, 'Active Ingredient' refers to (±)-*trans*-5-fluoro-1,2,3,3a,9,9a-hexahydro[1]benzopyrano[2,3-c]pyrrole hydrochloride. Other compounds of the invention may be formulated in similar fashion.

1. *Oral Capsule*

| | per capsule |
|---|---|
| Active Ingredient | 10 mg |
| Magnesium stearat | 0.5 mg |
| Anhydrous lactose | 90 mg |

Blend the active ingredient with the lactose and magnesium stearate. Fill the blend into appropriate size hard gelatin capsules (lock fitting type) on an automatic capsule filling machine.

2. *Oral Syrup*

| | per 5 ml dose |
|---|---|
| Active Ingredient | 10 mg |
| Sodium citrate | 25 mg |
| Citric acid | to pH 4.5 |
| Sunset yellow FCF (Dye) | 0.25 mg |
| Methyl hydroxybenzoate sodium | 5.0 mg |
| Propyl hydroxybenzoate sodium | 2.0 mg |
| Liquid orange flavour | qS |
| Sucrose | 3.50 g |
| Purified water | to 50 ml |

Dissolve the sucrose in a minimum quantity of water. Add a concentrated solution of sodium citrate with stirring and adjust the pH to 4.5 with citric acid. With continued stirring, add a 10% aqueous solution of the active ingredient, followed by a solution of the dye, a solution of the hydroxybenzoates and lastly the flavour. Adjust almost to volume with water and stir. Check the pH and adjust to 4.5 with citric acid if necessary. Make up to volume with water.

3. *Oral Tablet*

| (A) | per tablet |
|---|---|
| Active Ingredient | 10 mg |
| Polyvinylpyrrolidone | 4.0 mg |
| Sodium starch glycollate | 10.0 mg |
| Magnesium stearate | 2.0 mg |
| Lactose to tablet core weight to | 200 mg |

Blend the active ingredient with the lactose. Add a sufficient quantity of polyvinylpyrrolidone solution to produce a damp mass suitable for granulation. Prepare the granules and dry using a tray or fluid bed dryer. Pass through a sieve, blend with the remaining ingredients and compress into 8 mm diameter tablets on a tablet machine.

Film coat the tablet cores with hydroxypropyl methyl cellulose or similar film forming material, using either an aqueous or non-aqueous solvent system. A plasticizer and suitable colour may be included in the film coating solution.

| (B) | per tablet |
|---|---|
| Active Ingredient | 10 mg |
| Microcrystalline cellulose | 132.0 mg |
| Sodium starch glycollate | 5.0 mg |
| Sodium lauryl sulphate | 3.0 mg |

Sieve the active ingredient and the microcrystalline cellulose through a 40 mesh screen. Sieve the sodium starch glycollate and sodium lauryl sulphate through a 60 mesh screen. Blend the powders together in a suitable blender until homogeneous. Compress on apppropriate punches on an automatic tablet machine. The tablets may be covered in a thin polymer coat applied by the usual film coating techniques. A pigment may be included in the film coat.

**Claims for the Contracting States: BE CH DE FR GB IT LI LU NL SE**

1. Compounds of general formula (I):

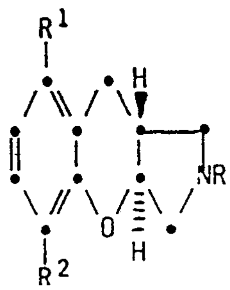

(I)

wherein

R is a hydrogen atom or a group selected from $C_{1-6}$ alkyl, $C_{1-6}$ alkyl substituted by $C_{3-7}$ cycloalkyl, $C_{3-6}$ alkenyl, $C_{3-6}$ alkynyl, $C_{3-7}$ cycloalkyl, and phen $C_{1-5}$ alkyl; and

$R^1$ and $R^2$, each independently represents a fluorine atom or a chlorine atom or one of $R^1$ and $R^2$ represents a hydrogen atom; and the physiologically acceptable salts thereof.

2. Compounds according to claim 1, wherein, in the general formula (I), R is a hydrogen atom.

3. Compounds according to claim 1, wherein, in the general formula (I), $R^1$ and/or $R^2$ represents a fluorine atom.

4. Compounds according to claim 1, wherein, in the general formula (I), R is a hydrogen atom, and one of $R^1$ and $R^2$ is a hydrogen atom and the other represents a fluorine atom.

5. (±)-*trans*-5-Fluoro-1,2,3,3a,9,9a-hexahydro[1]benzopyrano[2,3-c]pyrrole and its 3aS- and 3aR-isomers and the physiologically acceptable salts thereof.

6. Compounds according to any of claims 1 to 5 wherein the physiologically acceptable salts are selected from hydrochloride, hydrobromides, posphates, sulphates, citrates, tartrates, acetates, maleates and succinates.

7. Compounds according to claim 6, wherein the physiologically acceptable salts are the hydrochlorides.

8. A pharmaceutical composition comprising a compound of general formula (I) as defined in claim 1 and/or a physiologically acceptable salt thereof together with a physiologically acceptable carrier or excipient.

9. A process for preparing a compound of general formula (I) as defined in claim 1, or a physiologically acceptable salt thereof which comprises

(A) aminating a compound of general formula (II):

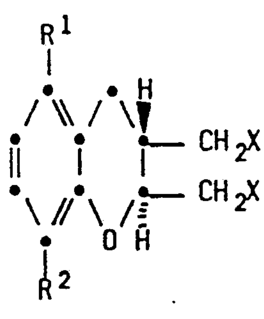

(II)

wherein X is a suitable leaving group and $R^1$ and $R^2$ are as defined in claim 1, with ammonia, aqueous ammonia or an amine of formula $RNH_2$, where R is as defined in claim 1; or

(B) in order to prepare a compound of general formula (I) where R represents a hydrogen atom, deprotecting a corresponding compound where R represents a protecting group, and, if necessary or desired, subjecting to the resulting compound to one or two further reactions comprising:

(C) (i) converting the resulting compound of general formula (I) or a salt thereof into another compound of general formula (I): and/or

(C) (ii) converting a compound of general formula (I) or a salt thereof into a physiologically acceptable salt thereof.

10. Compounds of formula (III):

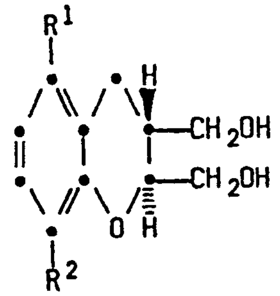

(III)

wherein $R^1$ and $R^2$ are as defined in claim 1.

**Claims for the Contracting States: AT GR ES**

1. A process for preparing a compound of general formula (I):

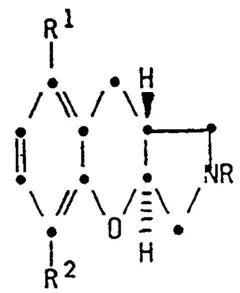

(I)

wherein

R is a hydrogen atom or a group selected from $C_{1-6}$ alkyl, $C_{1-6}$ alkyl substituted by $C_{3-7}$ cycloalkyl, $C_{3-6}$ alkenyl, $C_{3-6}$ alkynyl, $C_{3-7}$ cycloalkyl, and phen $C_{1-5}$ alkyl; and

$R^1$ and $R^2$, each independently represents a fluorine atom or a chlorine atom or one of $R^1$ and $R^2$ represents a hydrogen atom; or a physiologically acceptable salt thereof which comprises

(A) aminating a compound of general formula (II):

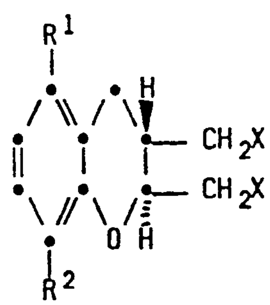

(II)

wherein X is a suitable leaving group and $R^1$ and $R^2$ are as defined in formula (I), with ammonia, aqueous ammonia or an amine of formula $RNH_2$, where R is as defined for formula (I); or

(B) in order to prepare a compound of general formula (I) where R represents a hydrogen atom, deprotecting a corresponding compound where R represents a protecting group, and, if necessary or desired, subjecting to the resulting compound to one or two further reactions comprising:

(C) (i) converting the resulting compound of general formula (I) or a salt thereof into another compound of general formula (I); and/or

(C) (ii) converting a compound of general formula (I) or a salt thereof into a physiologically acceptable salt thereof.

2. A process according to claim 1, wherein, in the general formula (I), R is a hydrogen atom.

3. A process according to claim 1, wherein, in the general formula (I), $R^1$ and/or $R^2$ represents a fluorine atom.

4. A process according to claim 1, wherein, in the general formula (I), R is a hydrogen atom, and one of $R^1$ and $R^2$ is a hydrogen atom and the other represents a fluorine atom.

5. A process according to claim 1, wherein the product is (±)-*trans*-5-fluoro-1,2,3,3a,9,9a-hexahydro[1]benzopyrano[2,3-c]pyrrole or its 3aS- and 3aR- isomers or a physiologically acceptable salt thereof.

6. A process according to claim 1, wherein in the step (C)(ii) the free base of formula (I) or a salt thereof is reacted with an acid selected from hydrogen chloride, hydrogen bromide, phosphoric acid, sulphuric acid, citric acid, tartaric acid, acetic acid, maleic acid and succinic acid.

7. A process according to claim 1, wherein amination is carried out at an elevated temperature in the presence of a base or in the presence of an excess of the amine $RNH_2$.

8. A process according to claim 1, wherein in process step (B) an arylmethyl protecting group is removed by hydrogenolysis or under acidic conditions or an acyl protecting group may be removed by hydrolysis.

9. A process for preparing a compound of formula (III):

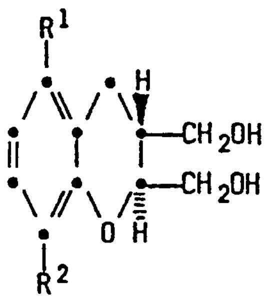

(III)

wherein $R^1$ and $R^2$ are as defined for formula (I) in claim 1, which comprises reducing an ester of formula (IV):

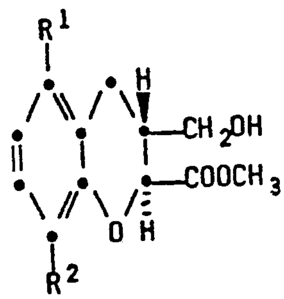

(IV)

wherein $R^1$ and $R^2$ are as defined for formula (I) in claim 1.

**Patentansprüche für die Vertragsstaaten: BE CH DE FR GB IT LI LU NL SE**

1. Verbindungen der allgemeinen Formel (I):

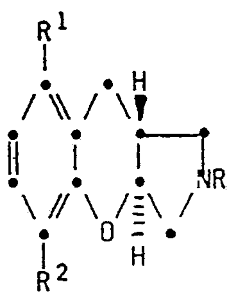

(I)

worin

R ein Wasserstoffatom oder eine Gruppe, ausgewählt aus $C_{1-6}$-Alkyl, $C_{1-6}$-Alkyl, das durch $C_{3-7}$-

Cycloalkyl substituiert ist, $C_{3-6}$-Alkenyl, $C_{3-6}$-Alkinyl, $C_{3-7}$-Cycloalkyl und Phen-$C_{1-5}$-alkyl ist; und

$R^1$ und $R^2$ unabhängig voneinander für ein Fluoratom oder ein Chloratom stehen oder einer der Substituenten $R^1$ und $R^2$ ein Wasserstoffatom darstellt, und die physiologisch annehmbaren Salze davon.

2. Verbindung nach Anspruch 1, dadurch gekennzeichnet, daß in der allgemeinen Formel (I) R ein Wasserstoffatom ist.

3. Verbindung nach Anspruch 1, dadurch gekennzeichnet, daß in der allgemeinen Formel (I) $R^1$ und/oder $R^2$ ein Fluoratom darstellen.

4. Verbindung nach Anspruch 1, dadurch gekennzeichnet, daß in der allgemeinen Formel (I) R ein Wasserstoffatom ist und einer der Substituenten $R^1$ und $R^2$ ein Wasserstoffatom ist und der andere ein Fluoratom ist.

5. (±)-trans-5-Fluor-1,2,3,3a,9,9a-hexahydro[1])benzopyrano[2,3-c]pyrrol und seine 3aS- und 3aR-Isomeren und die physiologisch annehmbaren Salze davon.

6. Verbindungen nach einem der Ansprüche 1 bis 5, dadurch gekennzeichnet, daß die physiologisch annehmbaren Salze aus Hydrochloriden, Hydrobromiden, Phosphaten, Sulfaten, Citraten, Tartraten, Acetaten, Maleaten und Succinaten ausgewählt sind.

7. Verbindung nach Anspruch 6, dadurch gekennzeichnet, daß die physiologisch annehmbaren Salze die Hydrochloride sind.

8. Pharmazeutisches Präparat, dadurch gekennzeichnet, daß es eine Verbindung de allgemeinen Formel (I) nach Anspruch 1 und/oder ein physiologisch annehmbaren Salz davon zusammen mit einem physiologisch annehmbaren Träger oder Exzipiens enthält.

9. Verfahren zur Herstellung einer Verbindung der allgemeinen Formel (I) nach Anspruch 1 oder eines physiologisch annehmbaren Salzes davon, dadurch gekennzeichnet, daß man

(A) eine Verbindung der allgemeinen Formel (II):

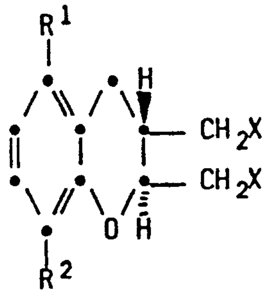

(II)

worin X eine geeignete Austrittsgruppe ist und $R^1$ und $R^2$ wie in Anspruch 1 definiert sind,

mit Ammoniak, wäßrigem Ammoniak oder einem Amin der Formel $RNH_2$, worin R wie in Anspruch 1 definiert ist, aminiert oder daß man

(B) zur Herstellung einer Verbindung der allgemeinen Formel (I), worin R ein Wasserstoffatom ist, von einer entsprechenden Verbindung, bei der R eine Schutzgruppe ist, die Schutzgruppe abspaltet und erforderlichenfalls oder gewünschtenfalls die resultierende Verbindung einer oder zwei weiteren Reaktionen, umfassend:

(C) (i) Umwandlung der resultierenden Verbindung der allgemeinen Formel (I) oder eines Salzes davon in eine andere Verbindung der allgemeinen Formel (I); und/oder

(C) (ii) Umwandlung einer Verbindung der allgemeinen Formel (I) oder eines Salzes davon in ein physiologisch annehmbares Salz davon, unterwirft.

10. Verbindungen der Formel (III):

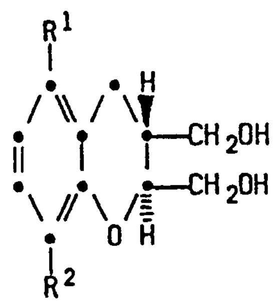

(III)

worin $R^1$ und $R^2$ wie in Anspruch 1 definiert sind.

**Patentansprüche für die Vertragsstaaten: AT GR ES**

1. Verfahren zur Herstellung einer Verbindung der allgemeinen Formel (I):

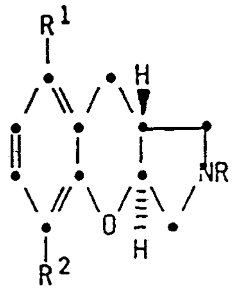

(I)

worin

R ein Wasserstoffatom oder eine Gruppe, ausgewählt aus $C_{1-6}$-Alkyl, $C_{1-6}$-Alkyl, das durch $C_{3-7}$-Cycloalkyl substituiert ist, $C_{3-6}$-Alkenyl, $C_{3-6}$-Alkinyl, $C_{3-7}$-Cycloalkyl und Phen-$C_{1-5}$-alkyl ist; und

$R^1$ und $R^2$ unabhängig voneinander für ein Fluoratom oder ein Chloratom stehen oder einer der Substituenten $R^1$ und $R^2$ ein Wasserstoffatom darstellt, oder eines physiologisch annehmbaren Salzes davon, dadurch gekennzeichnet, daß man

(A) eine Verbindung der allgemeinen Formel (II):

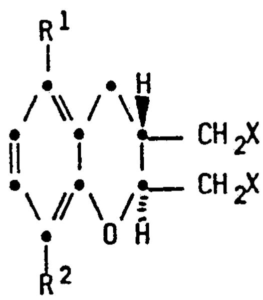

(II)

worin X eine geeignete Austrittsgruppe ist und $R^1$ und $R^2$ wie in Zusammenhang mit Formel (I) definiert sind,

mit Ammoniak, wäßrigem Ammoniak oder einem Amin der Formel $RNH_2$, worin R wie im Zusammenhang mit Formel (I) definiert ist, aminiert oder daß man

(B) zur Herstellung einer Verbindung der allgemeinen Formel (I), worin R ein Wasserstoffatom ist, von einer entsprechenden Verbindung, bei der R eine Schutzgruppe ist, die Schutzgruppe abspaltet und erforderlichenfalls oder gewünschtenfalls die resultierende Verbindung einer oder zwei weiteren Reaktionen, umfassend:

(C) (i) Umwandlung der resultierenden Verbindung der allgemeinen Formel (I) oder eines Salzes davon in eine andere Verbindung der allgemeinen Formel (I); und/oder

(C) (ii) Umwandlung einer Verbindung der allgemeinen Formel (I) oder eines Salzes davon in ein physiologisch annehmbares Salz davon, unterwirft.

2. Verfahren nach Anspruch 1, dadurch gekennzeichnet, daß in der allgemeinen Formel (I) R ein Wasserstoffatomen ist.

3. Verfahren nach Anspruch 1, dadurch gekennzeichnet, daß in der allgemeinen Formel (I) $R^1$ und/oder $R^2$ ein Fluoratom darstellen.

4. Verfahren nach Anspruch 1, dadurch gekennzeichnet, daß in der allgemeinen Formel (I) R ein Wasserstoffatom is und einer der Substituenten $R^1$ und $R^2$ ein Wasserstoffatom ist und der andere ein Fluoratom ist.

5. Verfahren nach Anspruch 1, dadurch gekennzeichnet, daß das Produkt (±)-trans-5-Fluor-1,2,3,3a,9,9a-hexahydro[1]benzopyrano[2,3-c]pyrrol oder seine 3aS- und 3aR-Isomeren oder ein physiologisch annehmbares Salze davon ist.

6. Verfahren nach Anspruch 1, dadurch gekennzeichnet, daß man in Stufe (C) (ii) die freie Base der Formel (I) oder ein Salze davon mit einer Säure ausgewählt aus Chlorwasserstoff, Bromwasserstoff, Phosphorsäure, Schwefelsäure, Zitronensäure, Weinsäure, Essigsäure, Maleinsäure und Bernsteinsäure, umsetzt.

7. Verfahren nach Anspruch 1, dadurch gekennzeichnet, daß man die Aminierung bei erhöhter Temperatur in Gegenwart einer Base oder in Gegenwart eines Überschusses des Amins $RNH_2$ durchführt.

8. Verfahren nach Anspruch 1, dadurch gekennzeichnet, daß in Verfahrenstufe (B) eine Arylmethyl-Schutzgruppe durch Hydrogenolyse oder unter sauren Bedingungen entfernt wird oder daß eine Acyl-Schutzgruppe durch Hydrolyse entfernt werden kann.

9. Verfahren zur Herstellung einer Verbindung der Formel (III):

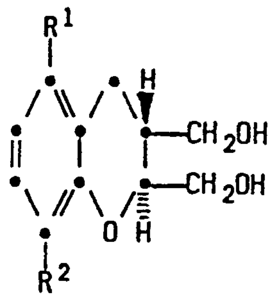

(III)

worin $R^1$ und $R^2$ wie im Zusammenhang mit Formel (I) in Anspruch 1 definiert sind, dadurch gekennzeichnet, daß man einen Ester der Formel (IV):

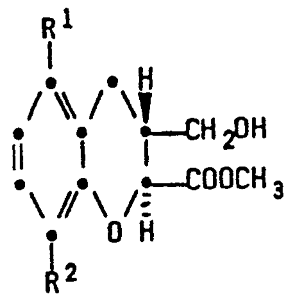

(IV)

worin $R^1$ und $R^2$ wie im Zusammenhang mit Formel (I) in Anspruch 1 definiert sind, reduziert.

**Revendications pour les Etats contractants: BE CH DE FR GB IT LI LU NL SE**

1. Composés de formule générale (I):

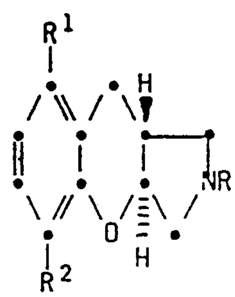

(I)

dans laquelle R représente un atome d'hydrogène, ou un groupe choisi parmi les groupes alkyle en $C_1$—$C_6$, alkyle en $C_1$—$C_6$ substitué avec un groupe cycloalkyle en $C_3$—$C_7$, alkényle en $C_3$—$C_6$, alkynyle en $C_3$—$C_6$, cycloalkyle en $C_3$—$C_7$, et phényl(alkyle en $C_1$—$C_5$); et $R^1$ et $R^2$ représentent chacun indépendamment, un atome de fluor ou un atome de chlore, ou l'un des groupes $R^1$ et $R^2$, représenté un atome d'hydrogène; ainsi que leurs sels physiologiquement acceptables.

2. Composés selon la revendication 1, dans la formule générale (I) desquels, R représente un atome d'hydrogène.

3. Composés selon la revendication 1, dans la formule générale (I) desquels, $R^1$ et/ou $R^2$, représentent des atomes de fluor.

4. Composés selon la revendication 1, dans la formule générale (I) desquels, R représente un atome d'hydrogène, et l'une des groupes $R^1$ et $R^2$, représente un atome d'hydrogène, et l'autre représente un atome de fluor.

5. (±)-*trans*-5-fluoro-1,2,3,3a,9,9a-hexahydro[1]benzopyrano[2,3-c]pyrrole, ses isomères 3aS- et 3aR- et leurs sels physiologiquement acceptables.

6. Composés selon l'une quelconque des revendications 1 à 5, les sels physiologiquement acceptables, étant choisis parmi les chlorhydrates, les bromhydrates, les phosphates, les sulfates, les citrates, les tartrates, les acétates, les maléates et les succinates.

7. Composés selon la revendication 6, les sels physiologiquement acceptables, étant les chlorhydrates.

8. Composition pharmaceutique, comprenant un composé de formule générale (I) tel que défini dans la

revendication 1, et/ou un sel physiologiquement acceptable de celui-ci, associé à un véhicule ou un excipient physiologiquement acceptable.

9. Procédé de préparation d'un composé de formule générale (I) tel que défini dans la revendication 1, ou d'un sel physiologiquement acceptable de celui-ci, dans lequel:

(A) on amine un composé de formule générale (II):

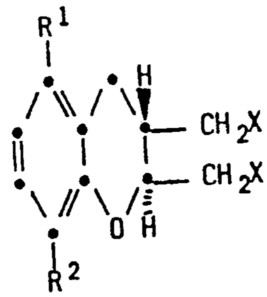

(II)

dans laquelle X représente un groupe partant approprié, et $R^1$ et $R^2$ sont tels que définis dans la revendication 1, avec de l'ammoniac, de l'ammoniac aqueux ou une amine de formule $RNH_2$ dans laquelle R est tel que défini dans la revendication 1; ou

(B) afin de préparer un composé de formule générale (I) dans laquelle R représente un atome d'hydrogène, on déprotège un composé correspondant dans lequel R représent un groupe protecteur, et si cela est nécessaire ou souhaitable, on soumet le composé résultant à une ou deux autres réactions, selon lesquelles:

(C) (i) on convertit le composé résultant de formule générale (I) ou un sel de celui-ci, en un autre composé de formule générale (I); et/ou

(C) (ii) on convertit un composé de formule générale (I) ou un sel de celui-ci, en un sel physiologiquement accepable de celui-ci.

10. Composés de formule (III):

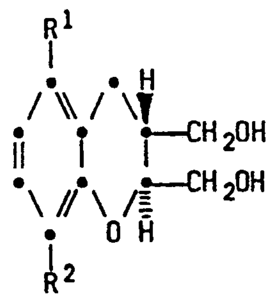

(III)

dans laquelle $R^1$ et $R^2$ sont tel que définis dans la revendication 1.

**Revendications pour les Etats contractants: AT GR ES**

1. Procédé de préparation d'un composé de formule générale (I):

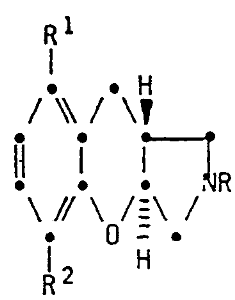

(I)

dans laquelle R représente un atome d'hydrogène, ou un groupe choisi parmi les groupes alkyle en $C_1$—$C_6$, alkyle en $C_1$—$C_6$ substitué avec un groupe cycloalkyle en $C_3$—$C_7$, alkényle en $C_3$—$C_6$, alkynyle en $C_3$—$C_6$, cycloalkyle en $C_3$—$C_7$, et phényl(alkyle en $C_1$—$C_5$); et $R^1$ et $R^2$ représentent indépendamment chacun, un atome de fluor ou un atome de chlore, ou l'un des groupes $R^1$ et $R^2$, représenté un atome d'hydrogène; ou un sel physiologiquement acceptable de celui-ci, dans lequel:

(A) on amine un composé de formule générale (II):

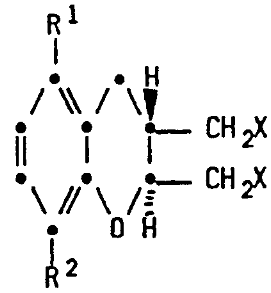

(II)

dans laquelle X représente un groupe partant approprié, et $R^1$ et $R^2$ sont tels que définis pour la formule (I), avec de l'ammoniac, de l'ammoniac aqueux ou une amine de formule $RNH_2$ dans laquelle R est el que défini pour la formule (I); ou

(B) afin de préparer un composé de formule générale (I) dans laquelle R représente un atome d'hydrogène, on déprotège un composé correspondant dans lequel R représent un groupe protecteur, et si cela est nécessaire ou requis, on soumet le composé résultant, à une ou deux autres réactions, selon lesquelles:

(C) (i) on convertit le composé résultant de formule générale (I) ou un sel de celui-ci, en un autre composé de formule générale (I); et/ou

(C) (ii) on convertit un composé de formule générale (I) ou un sel de celui-ci, en un sel physiologiquement accepable de celui-ci.

2. Procédé selon la revendication 1, dans lequel, dans la formule générale (I), R représente un atome d'hydrogène.

3. Procédé selon la revendication 1, dans lequel, dans la formule générale (I), $R^1$ et/ou $R^2$, représentent des atomes de fluor.

4. Procédé selon la revendication 1, dans lequel, dans la formule générale (I), R représente un atome d'hydrogène, et l'un des groupes $R^1$ et $R^2$, représente un atome d'hydrogène, et l'autre représente un atome de fluor.

5. Procédé selon la revendication 1, dans lequel le produit est le (±)-*trans*-5-fluoro-1,2,3,3a,9,9a-hexahydro[1]benzopyrano[2,3-c]pyrrole, ses isomerès 3aS- et 3aR- ou un physiologiquement acceptable de celui-ci.

6. Procédé selon la revendication 1, dans l'étape (C) (ii) duquel, on fait réagir la base libre de formule (I) ou un sel de celle-ci, avec un acide choisi parmi le chlorure d'hydrogène, le bromure d'hydrogène, l'acide phosphorique, l'acide sulfurique, l'acide citrique, l'acide tartrique, l'acide acétique, l'acide maléique et l'acide succinique.

7. Procédé selon la revendication 1, dans lequel l'amination est effectuée à une température élevée, en présence d'une base, ou en présence d'un excès de l'amine $RNH_2$.

8. Procédé selon la revendication 1, dans l'étape (B) duquel, on supprime un groupe arylméthyle protecteur, par hydrogénolyse, ou dans des conditions acides, ou un groupe acyle protecteur, peut être supprimé par hydrolyse.

9. Procédé de preparation d'un composé de formule générale (III):

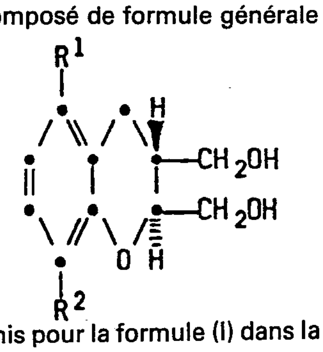

(III)

dans laquelle $R^1$ et $R^2$ sont tels que définis pour la formule (I) dans la revendication 1; dans lequel on réduit un ester de formule (IV):

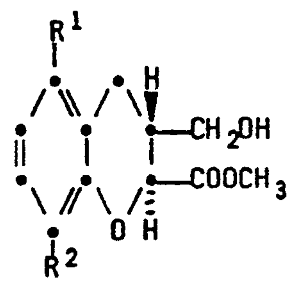

(IV)

dans laquelle $R^1$ et $R^2$ sont tels que définis pour la formule (I) dans la revendication 1.